(19) Europäisches Patentamt
European Patent Office
Office européen des brevets

(11) Publication number: **0 116 207**
**A2**

(12) # EUROPEAN PATENT APPLICATION

(21) Application number: 83307390.1

(22) Date of filing: 06.12.83

(51) Int. Cl.$^3$: **A 61 K 7/11**

(30) Priority: 15.12.82 GB 8235691

(43) Date of publication of application: 22.08.84 Bulletin 84/34

(84) Designated Contracting States: DE FR GB IT

(71) Applicant: BEECHAM GROUP PLC
Beecham House Great West Road
Brentford Middlesex(GB)

(72) Inventor: Elliott, Thomas Joseph
13 Hervey Close Finchley
London N3 2HG(GB)

(72) Inventor: Wisbey, Clive Alan
38 Pearscroft Court Fulham
London SW6 2BL(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Cosmetic formulation.**

(57) An aerosol hair-spray formulation, comprising a hair-holding resin dissolved in a solvent system, an aerosol propellant, and from 0.5 to 10% by weight of the formulation of at least one cyclic silicone of the general formula (I):

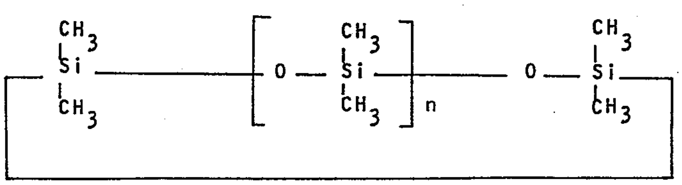

(I)

in which n is 1, 2 or 3, is provided which is useful in holding hair in place.

EP 0 116 207 A2

Croydon Printing Company Ltd.

# COSMETIC FORMULATION

The present invention relates to a cosmetic formulation, and in particular to an aerosol hair-spray formulation.

Aerosol hair-spray formulations containing a resin material dissolved in a solvent such as ethanol are well known. These formulations usually contain propellant material, such as a mixture of fluorocarbons, and are packed into a pressurised dispenser. When the formulation is applied to hair as a fine spray, the solvent evaporates leaving a film of resin on the hair which helps to hold the hair in place. One disadvantage of the use of such a formulation is that the hair tends to be hard to the touch and, hence, aesthetically unpleasing. Recently, attempts have been made to reduce the hard feel associated with resin based hair-sprays by incorporating plasticisers into the formulations. These plasticisers are able to soften the resin film on the hair to give a softer feel and, in addition, to prevent the film flaking off the hair. While some success has been achieved with the use of silicone plasticisers such as Silicone Glycol Polymer DC193, manufactured by Dow Corning Corp., the resulting increase in softness is accompanied by a decrease in 'holding power' which makes the formulations less effective at keeping hair in place.

It has now been found that by replacing all or a major part of the conventional plasticisers by certain cyclic silicone fluids, the desired soft feel can be retained together with an effective level of holding power.

According to the present invention, there is provided an aerosol hair-spray formulation, comprising a hair-holding resin dissolved in a solvent system, an aerosol propellant, and from 0.5 to 10% by weight of the formulation of at least one cyclic silicone of the general formula (I):

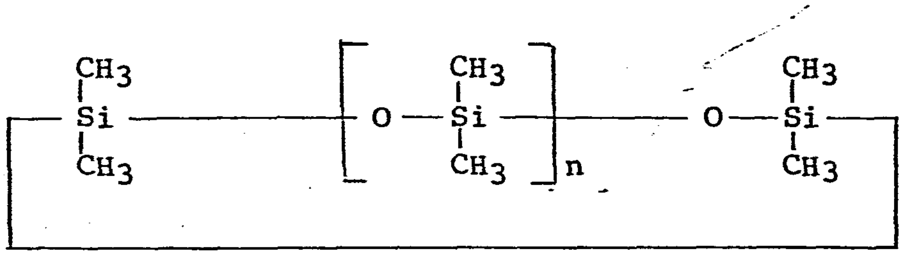

(I)

in which n is 1, 2 or 3.

Preferred cyclic silicones are dimethyl siloxane cyclic tetramer (n=2 in formula (I)) and dimethyl siloxane cyclic pentamer (n=3 in formula (I)).

The preferred weight range of cyclic silicones in the formulations is 0.5 to 5%, particularly 1 to 2%, by weight of formulation.

The hair-holding resin may be any of those which are conventionally used in aerosol hairspray formulations for holding hair in place.

Examples include copolymers of polyvinyl-pyrollidone/vinyl acetate, polyvinyl acetate/crotonic acid, and the butyl half ester of polyvinylmethyl ether/maleic anhydride.

Further examples include homopolymers of dimethylhydantoin/formaldehyde, and polyvinyl-pyrollidone; terpolymers such as vinyl acetate/crotonic acid/vinyl neodecanoate terpolymer; and acrylic resins such as octylacrylamide/acrylate/butylaminoethyl methacrylate polymer, and acrylate/acrylamide copolymer.

The above materials are commercially available as hairspray resins.

The proportion of resin in the formulation of the invention is preferably between about 0.5% and 5.0% by weight of the formulation.

The solvent system can be any of those conventionally used in resin hairspray formulations, but preferably comprises a $C_{1-6}$ alkanol, suitably ethanol. The proportion of solvent will vary according to the resin content of the formulation but is preferably from about 20% to 80% by weight of the formulation.

Any conventional aerosol propellant may be used in the present formulation, such as fluorocarbons or hydrocarbons or mixtures thereof, and the proportion will usually vary from about 80% to 20% by weight of the formulation.

If desired, the formulation of the invention may contain conventional plasticising materials such as the Silicone Glycol Polymer referred to above, and it has been found that good results are achieved with a relatively low level of conventional plasticiser in combination with a cyclic silicone of formula (I).

The formulations of the invention may additionally contain other conventional materials used in hairsprays, such as perfumes, lanolin or lanolin derivatives, spreading agents, surfactants and conditioning agents.

The aerosol formulations may be prepared and packed into suitable dispensers by conventional techniques well known in the art of aerosol technology.

The present invention also provides a method of holding human or non-human hair in place which comprises the application of an effective amount of a formulation of the present invention to the hair as an aerosol spray.

The invention will now be illustrated with reference to the following formulation Examples, and with reference to an experiment to measure the hair-'holding power' of hairsprays.

Method of Preparation

The formulations of the following Examples were prepared using the following procedure.

All ingredients except the propellant, were added in any order to the solvent system and stirred until completely homogenous. When an acidic hair resin is present the resin is neutralised by the addition of a sufficient amount of a base, preferablly an amine, such as 2-amino-2-methyl-propan-1-ol.

The formulation is packaged and the propellant added under pressure.

| | % w/w |
|---|---|
| **Example 1** | |
| Ultrahold 8A | 1.20 |
| 2-amino-2-methylpropan-1-ol | 0.10 |
| Silicone glycol polymer (DC 193) | 0.06 |
| Dimethyl siloxane cyclic tetramer | 1.00 |
| Ethanol | 37.64 |
| Propellant 11/12 (65:35) | 60.00 |
| | 100.00 |
| **Example 2** | |
| National 28-2930 | 1.80 |
| 2-amino-2-methylpropan-1-ol | 0.17 |
| Silicone glycol polymer (DC 193) | 0.10 |
| Dimethyl siloxane cyclic pentamer | 1.00 |
| Ethanol | 36.93 |
| Propellant 11/12 (65:35) | 60.00 |
| | 100.00 |
| **Example 3** | |
| Gantrez ES425 | 3.00 |
| 2-amino-2-methylpropan-1-ol | 0.06 |
| Dimethyl siloxane tetramer | 1.00 |
| Ethanol | 35.94 |
| Propellant 11/12 (65:35) | 60.00 |
| | 100.00 |
| **Example 4** | |
| Luviskol PVP K30 | 3.00 |
| Dimethyl siloxane cyclic tetramer | 1.00 |
| Ethanol | 36.00 |
| Propellant 11/12 (65:35) | 60.00 |
| | 100.00 |

0116207

## Example 5

| | % w/w |
|---|---|
| Ultrahold 8A | 0.94 |
| 2-amino-2-methylpropan-1-ol | 0.077 |
| Silicone glycol polymer (DC 193) | 0.02 |
| Perfume | 0.18 |
| Dimethyl siloxane cyclic pentamer (DC 345) | 1.0 |
| Denatured ethanol | 37.78 |
| Propellant 11 | 37.80 |
| Propellant 12 | 4.20 |
| n-Butane/iso-butane/propane | 18.00 |
| | 100.00 |

| | |
|---|---|
| Ultrahold 8A | - An acrylate/acrylamide copolymer manufactured by Ciba-Geigy. |
| National 28-2930 | - A polyvinyl acetate/crotonic acid copolymer manufactured by National Starch. |
| Gantrez ES425 | - Butyl half ester of polyvinyl methyl ether/maleic anhydride copolymer manufactured by G.A.F. |
| Luviskol PVP K30 | - A polyvinyl pyrollidone. |
| Propellant 11 | - Trichloromonofluoromethane. |
| Propellant 12 | - Dichlorodifluoromethane. |
| DC 345 | - A mixture of dimethyl siloxane cyclic pentamer with a small amount of tetramer marketed by Dow Corning Corp. |

Experimental Method

A standard test to measure the holding power of a hairspray formulation is the curl retention test.

In this test a standard swatch of hair approximately 15cm in length is curled on to a former so that its length is reduced to approximately 8cm. Whilst in the curled form it is given a 4 second spray from the aerosol hairspray under test and at the same time the tress on the former is rotated at 25rpm to ensure even distribution of the resin over the tress.

The tress (along with other tresses under test) is suspended from hooks in a cabinet maintained at 24°C and 90% relative humidity and its length measured. Changes in length of the hair produced by a progressive loss of curl retention are measured at various intervals of time.

Tests using two hairspray formulations of the invention and two formulations containing conventional plasticisers, but no cyclic silicones, were carried out using the above method.

The following formula A was used as the basic hairspray formulation and various silicone materials were incorporated at 1% by weight of the total formulation.

Formula A

| Ingredient | % w/w |
|---|---|
| Ultrahold 8A | 1.20 |
| 2-amino-2-methylpropan-1-ol | 0.10 |
| Silicone glycol polymer DC193 | 0.06 |
| Ethanol | to 40.00 |
| Propellant 11/12 (65:35) | 60.00 |

The following silicone materials were incorporated into separate samples of Formula A at 1% w/w

DC193 - A Silicone Glycol Polymer

DC556 - A polyphenyl methyl siloxane

DC344 - Dimethyl siloxane cyclic tetramer

DC345 - Dimethyl siloxane cyclic pentamer

The above silicone materials are marketed by Dow Corning Corp., DC193 and DC556 being conventional plasticisers currently recommended for plasticising hairspray resins.

Results

| Hairspray formulation | % Curl Retention with Time | | | |
|---|---|---|---|---|
| | Initial | 1h | 4h | 6h |
| 1) Formula A | 100 | 30.5 | 19.0 | 17.5 |
| 2) Formula A+1% DC344 | 100 | 41.0 | 28.5 | 26.0 |
| 3) Formula A+1% DC345 | 100 | 32.5 | 21.0 | 20.5 |
| 4) Formula A+1% DC193 | 100 | 20.5 | 11.0 | 6.5 |
| 5) Formula A+1% DC556 | 100 | 26.5 | 19.0 | 15.5 |

Conclusion

The results clearly show that the use of cyclic silicones provides increased holding power over formulations containing conventional plasticising materials.

In addition, formulations (2) and (3) were demonstrated by subjective testing, to produce a softer feel to the hair than that produced by formulations (1), (4) and (5).

CLAIMS

1. An aerosol hair-spray formulation comprising a hair-holding resin, dissolved in a solvent system, and an aerosol propellant, characterised in that it comprises from 0.5 to 10% by weight of the formulation of at least one cyclic silicone of formula (I);

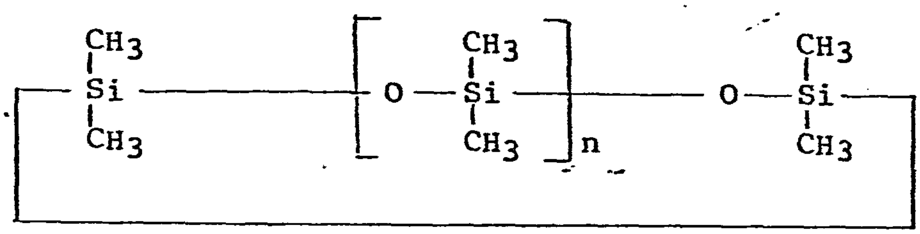

(I)

in which n is 1, 2 or 3.

2. A formulation as claimed in claim 1, wherein n is 2.

3. A formulation as claimed in claim 1, wherein n is 3.

4. A formulation as claimed in any one of claims 1 to 3, wherein the amount of cyclic silicone of formula (I) is from 0.5 to 5% by weight of the formulation.

5. A formulation as claimed in any one of claims 1 to 3, wherein the amount of cyclic silicone of formula (I) is from 1 to 2% by weight of the formulation.

6. A formulation as claimed in any one of claims 1 to 5, wherein the hair-holding resin comprises a polyvinylpyrollidone-vinyl acetate co-polymer, a polyvinyl acetate-crotonic acid co-polymer, a butyl half ester of polyvinylmethyl ether - maleic anhydride co-polymer, a homopolymer of dimethylhydantoin-formaldehyde, a homopolymer of polyvinylpyrollidone, a terpolymer of vinylacetate-crotonic acid-vinyl neodecanoate, a co-polymer of acrylate-acrylamide, and an octylacrylamide-acrylate-butylaminoethyl methacrylate polymer acrylic resin.

7. A formulation as claimed in any one of claims 1 to 6, wherein the amount of hair-holding resin is from 0.5 to 5% by weight of the formulation.

8. An aerosol formulation as claimed in any one of claims 1 to 7, wherein the solvent system comprises a $C_{1-6}$ alkanol.

9. A formulation as claimed in claim 8, wherein the solvent system comprises ethanol.

10. A formulation as claimed in claim 1, wherein the hair-holding resin comprises an acrylate-acrylamide co-polymer, n is 3, and which further comprises a silicone glycol polymer plasticiser.